# EUROPEAN PATENT APPLICATION

(11) **EP 3 476 377 A1**
(43) Date of publication of application: **01.05.2019**
(21) Application number: 17814410.1
(22) Date of filing: 23.01.2017
(51) Int. Cl.: A61F 13/537

(54) **ABSORBENT CORE WITH EFFICIENT FLOW GUIDE PROPERTY AND BREATHABILITY, AND METHOD FOR MANUFACTURING SAME**

(30) Priority: 23.06.2016 CN 201610458331
(71) Applicant: Hunan Cosom Care Products Co., Ltd., Changsha, Hunan 410600 (CN)
(72) Inventor: QIN, Xujun, Changsha Hunan 410600 (CN); TANG, Liang, Changsha Hunan 410600 (CN); GAN, Yi, Changsha Hunan 410600 (CN)
(74) Representative: Coles, Andrea Birgit
(86) International application number: PCT/CN2017/072158
(87) International publication number: WO 2017/219670

(57) **Abstract**

An absorbent core with an efficient flow guide property and breathability, and method for manufacturing same. The absorbent core comprises a surface layer (4), a bottom layer (5), and an absorbent layer between the surface layer (4) and the bottom layer (5); the absorbent layer comprises a non-woven fabric layer (6) attached to the lower surface of the surface layer (4); a super absorbent polymer is uniformly scattered in the non-woven fabric layer (6); the surface layer (4) and the non-woven fabric layer (6) are attached to each other to form a composite layer; a flow guide groove (7) is formed on the composite layer and divides the absorbent core into a plurality of absorbent regions; the composite layer with the flow guide groove (7) is connected with the upper surface of the bottom layer (5); a super absorbent polymer is uniformly scattered between the bottom surface of the non-woven fabric layer (6) and the upper surface of the bottom layer (5). According to the absorbent core, the degree of plasticization of the non-woven fabric layer can be maximally reduced, the softness and comfort are improved, and the touch feeling is improved. Super absorbent polymers with different properties are matchedly scattered between layers of the absorbent core, and obvious protrusions would be formed after liquid is absorbed by the absorbent core; the absorbent core feels soft and comfortable and is good in breathability, high in absorbed liquid spreading speed, and small in skin contact area.

## Description

### Field of the Invention

The present invention belongs to the field of sanitary articles, and particularly relates to an absorbent core with efficient flow guide property and breathability and a manufacturing method thereof.

### Background of the Invention

Currently known absorbent cores for treating human body excrement can be roughly divided into two types.

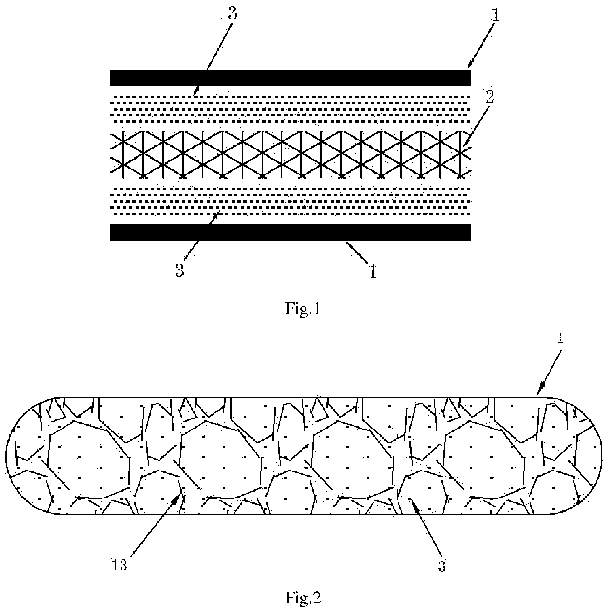

One is a composite core, as shown in Fig. 1, generally consisting of two outermost layers of airlaid paper 1, a non-woven fabric middle layer 2 and two layers of SAP 3 (super absorbent polymer).

The other is a cotton core, as shown in Fig. 2, consisting of airlaid paper 1 or hydrophilic cloth wrapping fluff pulp 13 and SAP 3.

In addition to the above-mentioned common structures, some manufacturers make fine adjustments on the structure of the absorber, such as adding a layer of wood pulp to the structure of Fig. 1, and embossing a pattern on the cotton core of Fig. 2 by using a grinding wheel.

The above-mentioned core is planar after absorbing liquid and has a large contact area with the skin, and the amount of SAP that can be added is limited. Adding a large amount of SAP is liable to cause sliding of SAP and obvious graininess, and the core is prone to layering and stacking by sliding after absorbing liquid.

A product using the absorbent core with the above structure has the following drawbacks during actual use:
1. As shown in Fig. 1, when a large amount of SAP needs to be added to this type of core, the usage amount of hot melt adhesive needs to be increased, otherwise, the large amount of SAP is liable to move, resulting in uneven distribution, and too much hot melt adhesive will cause obvious reduction in the absorption speed and the breathability, as well as stiff hand feeling.
2. As shown in Figs. 1 and 2, the above mentioned two types of core bodies are planar after absorbing liquid and are extended even if patterns are embossed on the core bodies, so that the contact area between the surface layer of the product and the skin is large, the air on the surface of the skin can not fully circulate and form convection, and the harmful gas volatilized from the liquid is likely to cause harm to the skin, inducing various skin diseases.
3. As shown in Fig. 1, this type of core has a weak liquid absorption diffusion effect, a low secondary absorption speed, and a low core utilization rate.
4. As shown in Fig. 2, this type of core expands in volume after the SAP absorbs liquid, and the particles are mobile. Even if meshes or streaks are pressed on the absorber, the embossing is propped up after the liquid absorption, such that the positions of the SAP particles cannot be fixed. In the case of a slight external force, the SAP absorbing liquid is easily displaced, resulting in local accumulation into a mass and formation of faults in other parts, which not only affects the secondary liquid absorption effect, but also affects the wearing comfort.
5. As shown in Fig. 2, this type of core generally uses a large amount of fluff pulp, and after liquid absorption, most of the liquid is instantaneously captured by the fluff pulp and is difficult to spread to both ends, which is likely to cause local expansion and bulge of the core. In addition to the proneness to fracture, water is not locked firmly, which is liable to cause liquid reverse osmosis, and the liquid easily volatilizes to harm the skin or affect the skin tactile sensation.

### Summary of the Invention

In view of the above problems, the present invention aims at providing an absorbent core which is capable of maximally improving the flow guide property and reducing the degree of plasticization of the non-woven fabric, and which is soft, comfortable and good in breathability.

The technical solution of the present invention to solve the problem is as follows:
an absorbent core with efficient flow guide property and breathability includes a surface layer, a bottom layer and an absorbent layer between the surface layer and the bottom layer; the absorbent layer includes a non-woven fabric layer attached to the lower surface of the surface layer, and a super absorbent polymer is uniformly scattered in the non-woven fabric layer;
the surface layer is attached to the non-woven fabric layer to form a composite layer, a flow guide groove is formed in the composite layer, and the flow guide groove divides the core into a plurality of absorbent regions;
the composite layer with the flow guide groove is connected with the upper surface of the bottom layer;
a super absorbent polymer is uniformly scattered between the lower surface of the non-woven fabric layer and the upper surface of the bottom layer.

In the above solution, the existing planar absorbent core is improved into a three-dimensional absorbent core, the core is divided into a plurality of absorbent regions by the flow guide groove, the core expands to form a mountain-like shape after the first time liquid absorption, and the liquid is naturally guided downward under the action of gravitational potential energy during the second time liquid absorption, thereby accelerating the liquid conduction and absorption and effectively improving the secondary absorption speed. The higher protrusion formed after the liquid absorption reduces the contact area of the surface layer and the skin and improves the breathability.

Another benefit of dividing the core into a plurality of absorbent regions is that SAP can be located in a relatively small space to prevent the SAP from shifting. In this way, more SAP can be added without using a large amount of hot melt adhesive, and no graininess is generated.

As more SAP is added between the lower surface of the non-woven fabric layer and the upper surface of the bottom layer, it is beneficial for the multiple-time absorption of the core.

Further, the non-woven fabric layer includes a first non-woven fabric layer and a second non-woven fabric layer located below the first non-woven fabric layer, a first super absorbent polymer is uniformly scattered in the first non-woven fabric layer, a second super absorbent polymer is uniformly scattered in the second non-woven fabric layer, and a third super absorbent polymer is uniformly scattered between the lower surface of the second non-woven fabric layer and the bottom layer; and
an air passage is formed between the first non-woven fabric layer and the second non-woven fabric layer.

Prior to liquid absorption, the air passage functions for ventilating and preventing stuffiness during wearing, and can provide a sufficient expansion space for the SAP after liquid absorption.

Preferably, the first, second, and third super absorbent polymers have different model numbers, and the liquid absorption speeds of the first, second, and third super absorbent polymers gradually increase successively to form a gradient difference while the liquid absorption speeds increase from top to bottom.

The SAP of different model numbers are placed in multiple layers, thereby reducing the pile-like graininess of the SAP due to movement, the position and amount of SAP can be selected according to the characteristics of SAP to adjust the absorption speed, the dryness and the like of the core, and the designability of the core performance is enhanced. Therefore, the overall utilization rate of the core is improved, and the absorption speed and the multiple-time liquid absorption effect of the core are remarkably improved.

Specifically, the flow guide groove is S-shaped or linear.

The attachment manner of the surface layer and the non-woven fabric layer is one or more of hot rolling, ultrasonic bonding, seaming and adhesive bonding.

The connection mode between the composite layer with the flow guide groove and the bottom layer is adhesive bonding; and

The flow guide groove is made by thermal bonding.

At the same time, the present invention further provides a manufacturing method of the absorbent core with efficient flow guide property and breathability in the above solution, which is characterized by including the following steps:
(a) uniformly scattering the super absorbent polymer in the voids of the non-woven fabric layer, and attaching the non-woven fabric layer to the surface layer to form the composite layer, wherein the attachment manner is one or more of hot rolling, ultrasonic bonding, seaming and adhesive bonding;
(b) preheating the composite layer in step (a) through a preheating device to 30-150 °C;
(c) processing the flow guide groove on the preheated composite layer by means of thermal bonding;
(d) uniformly scattering the super absorbent polymer on the lower surface of the composite non-woven fabric layer, and attaching the lower surface to the bottom layer via adhesive bonding; and
(e) performing ultrasonic slitting or hot slitting, and then performing coiling.

The focus of the above method is that the surface layer, the non-woven fabric layer and the bottom layer are not thermally bonded together, but some of the layers are firstly composited because bonding all the layers together needs a higher temperature and more heat, which causes the non-woven fabric layer to be severely plasticized, resulting in poor hand feeling.

The purpose of preheating is to reduce the temperature difference during the hot rolling, better reduce the degree of plasticization and improve the hand feeling.

The ultrasonic slitting or hot slitting is used for integrating the materials of the cut edges, reducing the possibility of the SAP leaking from the sides of the core, and eliminating the SAP leakage during the use of the finished product, and ensuring soft hand feeling at the same time.

In a preferred solution, the non-woven fabric layer includes a first non-woven fabric layer and a second non-woven fabric layer located on the lower surface of the first non-woven fabric layer, a first super absorbent polymer is uniformly scattered in the first non-woven fabric layer, a second super absorbent polymer is uniformly scattered in the second non-woven fabric layer, and a third super absorbent polymer is uniformly scattered between the lower surface of the second non-woven fabric layer and the bottom layer; and
correspondingly, in step (a), the step of attaching the non-woven fabric layer to the surface layer to form the composite layer can be specifically decomposed into:
(a1) uniformly scattering the first super absorbent polymer in the voids of the first non-woven fabric layer, and attaching the first non-woven fabric layer to the surface layer; and
(a2) uniformly scattering the a second super absorbent polymer in the voids of the second non-woven fabric layer, and stacking the second non-woven fabric layer and the first non-woven fabric layer;
the surface layer, the first non-woven fabric layer and the second non-woven fabric layer constitute the composite layer in the step (a), and an air passage is formed between the first non-woven fabric layer and the second non-woven fabric layer by a glue-free process; and
the first, second, and third super absorbent polymers have different model numbers, and the liquid absorption speeds of the first, second, and third super absorbent polymers gradually increase successively to form a gradient difference while the liquid absorption speeds increase from top to bottom.

Specifically, the preheating mode in the step (b) is one or more of infrared, hot air and ultrasonic waves, and the heating temperature is 80 °C.

The flow guide groove in the step (c) is processed by hot rolling, the surface temperature of a lower roll is lower than the surface temperature of an upper roll, the surface temperature of the upper roll is 80-180 °C, and the surface temperature of the lower roll is 30 to 90 °C, and the rolling speed is 50-150m/min.

In the prior art processing process, the upper and lower rolls have the same surface temperature, in the method of the present invention, the surface temperature of the lower roll is reduced, so that the lower surface material of the composite layer is plasticized to a small extent, and the softness of the entire core can be improved.

### Significant Effects of the Present Invention:

1. In the manufacturing process of the core, the surface layer, the non-woven layer and the bottom layer are not thermally bonded together, but some of the layers are firstly composited, then are pre-heated, then are thermally bonded, and finally are glued, so that the degree of plasticization of the non-woven fabric layer can be maximally reduced, the softness and comfort are improved, and the touch feeling is improved.
2. With the design of the flow guide groove, a stable air passage is formed on the surface of the core, which accelerates the rapid diffusion of the liquid along the passage and improves the overall utilization rate of the core at the same time.
3. The SAP is fed in multiple layers, so that the receiving capacity of the core is greatly improved, and the material of the middle part adopts an glue-free bonding process, so that the overall sizing amount of the core is significantly reduced, which is more conducive to liquid infiltration and effectively improving the absorption speed.
4. The layered feeding of the SAP in the core makes the matching form diversified, and the model number and the application amount of each layer SAP can be comprehensively designed from the aspects of water saturation, liquid permeability, amount of reverse osmosis and the like, so that the designability of the core performance is greatly improved.
5. The core uses no fluff pulp, most of the liquid is absorbed by the SAP at the beginning, and a small part of the liquid is temporarily stored by fiber non-woven fabric or airlaid paper, and then is entirely absorbed and locked by the SAP. Most of the harmful gases that can be volatilized are volatilized outward through a breathable film and the nonwoven fabric on the bottom layer, and very little harmful gas is volatilized from the surface layer of the absorber, so that the harm to the skin is reduced to the minimum.

### Brief Description of the Drawings

The present invention is further illustrated below in combination with the drawings.
Fig. 1 is a structural diagram of an absorbent core in the prior art.
Fig. 2 is a structural diagram of another absorbent core in the prior art.
Fig. 3 is a top view of an absorbent core in embodiment 1 of the present invention.
Fig. 4 is a sectional view of the absorbent core in embodiment 1 of the present invention.
Fig. 5 is a process block diagram of the absorbent core in embodiment 1 of the present invention.
Fig. 6 is a sectional view of an absorbent core in embodiment 2 of the present invention.
Fig. 7 is a process block diagram of the absorbent core in embodiment 2 of the present invention.
Fig. 8 is a top view of an absorbent core in embodiment 3 of the present invention.

Reference signs: 1-airlaid paper, 2-non-woven fabric middle layer, 3-SAP, 4-surface layer, 5-bottom layer, 6- non-woven fabric layer, 7-flow guide groove, 9-first super absorbent polymer, 10-second super absorbent polymer, 11-third super absorbent polymer, 12-air passage, 13-fluff pulp, 61-first non-woven fabric layer, and 62-second non-woven fabric layer.

### Detailed Description of the Embodiments

### Embodiment 1

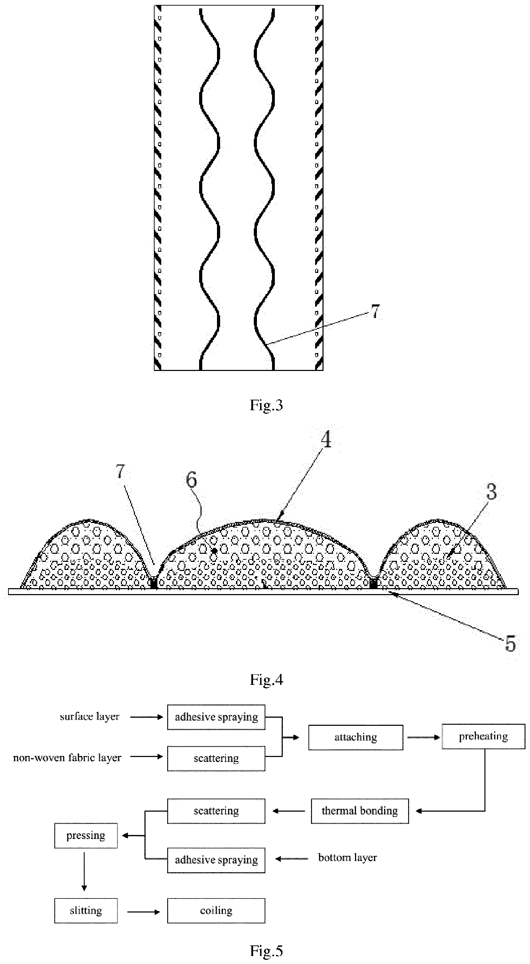

As shown in Figs. 3 to 5, an absorbent core with efficient flow guide property and breathability includes a surface layer 4, a bottom layer 5 and an absorbent layer between the surface layer 4 and the bottom layer 5. The absorbent layer includes a non-woven fabric layer 6 attached to the lower surface of the surface layer 4, and a super absorbent polymer is uniformly scattered in the non-woven fabric layer 6.

The surface layer 4 is attached to the non-woven fabric layer 6 to form a composite layer, a s-shaped flow guide groove 7 is formed in the composite layer, and the flow guide groove 7 divides the core into a plurality of absorbent regions.

The composite layer with the flow guide groove 7 is connected with the upper surface of the bottom layer 5.

A super absorbent polymer is uniformly scattered between the lower surface of the non-woven fabric layer 6 and the upper surface of the bottom layer 5.

The attachment manner of the surface layer 4 and the non-woven fabric layer 6 is one or more of hot rolling, ultrasonic bonding, seaming and adhesive bonding.

The connection mode between the composite layer with the flow guide groove 7 and the bottom layer 5 is adhesive bonding. The flow guide groove 7 is made by thermal bonding.

Correspondingly, the embodiment further provides a manufacturing method of the absorbent core with efficient flow guide property and breathability, which is characterized by including the following steps:
(a) uniformly scattering the super absorbent polymer in the voids of the non-woven fabric layer 6, and attaching the non-woven fabric layer 6 to the surface layer 4 to form the composite layer, wherein the attachment manner is one or more of hot rolling, ultrasonic bonding, seaming and adhesive bonding;
(b) preheating the composite layer in step (a) through a preheating device to 30-150 °C;
(c) processing the flow guide groove 7 on the preheated composite layer by means of thermal bonding;
(d) uniformly scattering the super absorbent polymer on the lower surface of the composite non-woven fabric layer 6, and attaching the lower surface to the bottom layer 5 via adhesive bonding; and
(e) performing ultrasonic slitting or hot slitting, and then performing coiling.

The preheating mode in the step (b) is one or more of infrared, hot air and ultrasonic waves, and the heating temperature is 80 °C.

The flow guide groove 7 in the step (c) is processed by hot rolling, the surface temperature of a lower roll is lower than the surface temperature of an upper roll, the surface temperature of the upper roll is 80-180 °C, and the surface temperature of the lower roll is 30 to 90 °C, and the rolling speed is 50-150m/min.

In the above-mentioned method, the surface layer, the non-woven fabric layer and the bottom layer are not thermally bonded together, but some of the layers are firstly composited because bonding all the layers together needs a higher temperature and more heat, which causes the non-woven fabric layer to be severely plasticized, resulting in poor hand feeling.

The purpose of preheating is to reduce the temperature difference during the hot rolling, better reduce the degree of plasticization and improve the hand feeling.

The flow guide groove 7 divides the core into a plurality of absorbent regions, the core expands to form a mountain-like shape after the first time liquid absorption, and the liquid is naturally guided downward under the action of gravitational potential energy during the second time liquid absorption, thereby accelerating the liquid conduction.

Another benefit of dividing the core into a plurality of absorbent regions is that the SAP can be located in a relatively small space to prevent the SAP from shifting. In this way, more SAP can be added without using a large amount of hot melt adhesive, and no graininess is generated.

As more SAP is added between the lower surface of the non-woven fabric layer 6 and the upper surface of the bottom layer 5, it is beneficial for the multiple-time absorption of the core.

The ultrasonic slitting or hot slitting is used for integrating the materials of the cut edges, reducing the possibility of the SAP leaking from the sides of the core, and eliminating the SAP leakage during the use of the finished product, and ensuring soft hand feeling at the same time.

### Embodiment 2

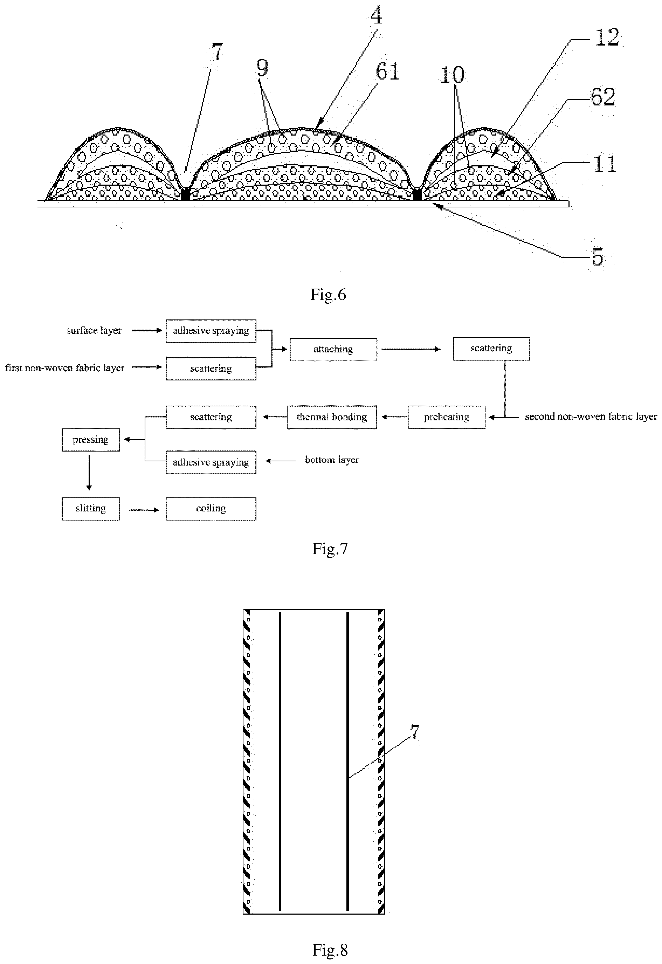

As shown in Figs. 6 to 7, embodiment 1 is repeated except for the difference that the non-woven fabric layer 6 includes a first non-woven fabric layer 61 and a second non-woven fabric layer 62 located below the first non-woven fabric layer 61. A first super absorbent polymer 9 is uniformly scattered in the first non-woven fabric layer 61. A second super absorbent polymer 10 is uniformly scattered in the second non-woven fabric layer 62. A third super absorbent polymer 11 is uniformly scattered between the lower surface of the second non-woven fabric layer 62 and the bottom layer 5.

Correspondingly, in step (a), the step of attaching the non-woven fabric layer 6 to the surface layer 4 to form the composite layer can be specifically decomposed into:
(a1) uniformly scattering the first super absorbent polymer 9 in the voids of the first non-woven fabric layer 61, and attaching the first non-woven fabric layer 61 to the surface layer 4; and
(a2) uniformly scattering the second super absorbent polymer 10 in the voids of the second non-woven fabric layer 62, and stacking the second non-woven fabric layer 62 and the first non-woven fabric layer 61;
the surface layer 4, the first non-woven fabric layer 61 and the second non-woven fabric layer 62 constitute the composite layer in the step (a), and an air passage 12 is formed between the first non-woven fabric layer 61 and the second non-woven fabric layer 62 by a glue-free process. Prior to liquid absorption, the air passage functions for ventilating and preventing stuffiness during wearing, and can provide a sufficient expansion space for the SAP after liquid absorption.

The first, second, and third super absorbent polymers have different model numbers, and the liquid absorption speeds of the first, second, and third super absorbent polymers gradually increase successively to form a gradient difference while the liquid absorption speeds increase from top to bottom.

The SAP of different model numbers are placed in multiple layers, thereby reducing the pile-like graininess of the SAP due to movement, the position and amount of SAP can be selected according to the characteristics of SAP to adjust the absorption speed, the dryness and the like of the core, and the designability of the core performance is enhanced.

### Embodiment 3

As shown in Fig. 8, embodiment 1 or 2 is repeated except for the difference that the flow guide groove 7 is linear.

## Claims

1. An absorbent core with efficient flow guide property and breathability, comprising a surface layer (4), a bottom layer (5) and an absorbent layer between the surface layer (4) and the bottom layer (5), wherein the absorbent layer comprises a non-woven fabric layer (6) attached to the lower surface of the surface layer (4), and a super absorbent polymer is uniformly scattered in the non-woven fabric layer (6);
the surface layer (4) is attached to the non-woven fabric layer (6) to form a composite layer, a flow guide groove (7) is formed in the composite layer, and the flow guide groove (7) divides the core into a plurality of absorbent regions;
the composite layer with the flow guide groove (7) is connected with the upper surface of the bottom layer (5);
a super absorbent polymer is uniformly scattered between the lower surface of the non-woven fabric layer (6) and the upper surface of the bottom layer (5).

2. The absorbent core with efficient flow guide property and breathability according to claim 1, wherein the non-woven fabric layer (6) comprises a first non-woven fabric layer (61) and a second non-woven fabric layer (62) located below the first non-woven fabric layer (61), a first super absorbent polymer (9) is uniformly scattered in the first non-woven fabric layer (61), a second super absorbent polymer (10) is uniformly scattered in the second non-woven fabric layer (62), and a third super absorbent polymer (11) is uniformly scattered between the lower surface of the second non-woven fabric layer (62) and the bottom layer (5); and
an air passage (12) is formed between the first non-woven fabric layer (61) and the second non-woven fabric layer (62).

3. The absorbent core with efficient flow guide property and breathability according to claim 2, wherein the liquid absorption speeds of the first, second, and third super absorbent polymers gradually increase successively to form a gradient difference while the liquid absorption speeds increase from top to bottom.

4. The absorbent core with efficient flow guide property and breathability according to claim 1, wherein the flow guide groove (7) is S-shaped or linear.

5. The absorbent core with efficient flow guide property and breathability according to claim 1, wherein the attachment manner of the surface layer (4) and the non-woven fabric layer (6) is one or more of hot rolling, ultrasonic bonding, seaming and adhesive bonding.

6. The absorbent core with efficient flow guide property and breathability according to claim 1, wherein the connection mode between the composite layer with the flow guide groove (7) and the bottom layer (5) is adhesive bonding;
and the flow guide groove (7) is made by thermal bonding.

7. A manufacturing method of the absorbent core with efficient flow guide property and breathability according to claim 1, comprising the following steps:
(a) uniformly scattering the super absorbent polymer in the voids of the non-woven fabric layer (6), and attaching the non-woven fabric layer (6) to the surface layer (4) to form the composite layer, wherein the attachment manner is one or more of hot rolling, ultrasonic bonding, seaming and adhesive bonding;
(b) preheating the composite layer in step (a) through a preheating device to 30-150 °C;
(c) processing the flow guide groove (7) on the preheated composite layer by means of thermal bonding;
(d) uniformly scattering the super absorbent polymer on the lower surface of the composite non-woven fabric layer (6), and attaching the lower surface to the bottom layer (5) via adhesive bonding; and
(e) performing ultrasonic slitting or hot slitting, and then performing coiling.

8. The manufacturing method according to claim 7, wherein the non-woven fabric layer (6) comprises a first non-woven fabric layer (61) and a second non-woven fabric layer (62) located below the first non-woven fabric layer (61), a first super absorbent polymer (9) is uniformly scattered in the first non-woven fabric layer (61), a second super absorbent polymer (10) is uniformly scattered in the second non-woven fabric layer (62), and a third super absorbent polymer (11) is uniformly scattered between the lower surface of the second non-woven fabric layer (62) and the bottom layer (5); and
correspondingly, in step (a), the step of attaching the non-woven fabric layer (6) to the surface layer (4) to form the composite layer can be specifically decomposed into:
(a1) uniformly scattering the first super absorbent polymer (9) in the voids of the first non-woven fabric layer (61), and attaching the first non-woven fabric layer (61) to the surface layer (4); and
(a2) uniformly scattering the second super absorbent polymer (10) in the voids of the second non-woven fabric layer (62), and stacking the second non-woven fabric layer (62) and the first non-woven fabric layer (61);
the surface layer (4), the first non-woven fabric layer (61) and the second non-woven fabric layer (62) constitute the composite layer in the step (a), and an air passage (12) is formed between the first non-woven fabric layer (61) and the second non-woven fabric layer (62) by a glue-free process; and
the liquid absorption speeds of the first, second, and a third super absorbent polymer gradually increase successively to form a gradient difference while the liquid absorption speeds increase from top to bottom.

9. The manufacturing method according to claim 7 or 8, wherein the preheating mode in the step (b) is one or more of infrared, hot air and ultrasonic waves, and the heating temperature is 80 °C.

10. The manufacturing method according to claim 7 or 8, wherein the flow guide groove (7) in the step (c) is processed by hot rolling, the surface temperature of a lower roll is lower than the surface temperature of an upper roll, the surface temperature of the upper roll is 80-180 °C, and the surface temperature of the lower roll is 30 to 90 °C, and the rolling speed is 50-150m/min.
